# EUROPÄISCHE PATENTSCHRIFT

(11) **EP 2 717 845 B1**
(45) Veröffentlichungstag und Bekanntmachung des Hinweises auf die Patenterteilung: **01.03.2017**
(21) Anmeldenummer: 12725816.8
(22) Anmeldetag: 06.06.2012
(51) Int. Cl.: A61K 8/73, A61K 8/81, A61Q 5/06

(54) **STYLINGMITTEL MIT HÖCHSTEM HALT**
STYLING AGENTS HAVING MAXIMUM HOLD
AGENT DE MISE EN FORME À TENUE MAXIMALE

(30) Priorität: 10.06.2011 DE 102011077364
(43) Veröffentlichungstag der Anmeldung: 16.04.2014
(73) Patentinhaber: Henkel AG & Co. KGaA, 40589 Düsseldorf (DE)
(72) Erfinder: KNAPPE, Thorsten, 22869 Schenefeld (DE); FLODROP VAN, Helga, 21075 Hamburg (DE)
(86) Internationale Anmeldenummer: PCT/EP2012/060720
(87) Internationale Veröffentlichungsnummer: WO 2012/168311

(56) Entgegenhaltungen:
- WO-A2-2008/098717
- WO-A2-2010/020503
- US-A1- 2006 078 507
- JENNIFER REICHL COLLIN ET AL: "Aculyn TM 88 rheology modifier", RESEARCH DISCLOSURE, MASON PUBLICATIONS, HAMPSHIRE, GB, Nr. 494011, 1. Juni 2005 (2005-06-01), Seiten 1-12, XP002661603, ISSN: 0374-4353 [gefunden am 2005-05-27]
- MIAO WANG OF THE ROHM AND HAAS COMPANY ET AL: "Mousse formulations containing Acudyne(TM) DHR or Acudyne(TM) 180 hair fixative polymer and Aculyn(TM) 88 rheology modifier", RESEARCH DISCLOSURE, MASON PUBLICATIONS, HAMPSHIRE, GB, Bd. 510, Nr. 27, 1. Oktober 2006 (2006-10-01), Seite 1297, XP007136706, ISSN: 0374-4353

## Beschreibung

Die vorliegende Erfindung betrifft Mittel zur Haarbehandlung, enthaltend eine Kombination mindestens eines speziellen vernetzten amphiphilen, anionischen Polymers mit mindestens einem weiteren speziellen unvernetzten amphiphilen, anionischen Polymer und mindestens einem dritten Polymer, die Verwendung dieser Mittel zur temporären Verformung und/oder zur Pflege keratinhaltiger Fasern und Haargele auf Basis dieser Mittel.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Produkte mit hohen Haltegraden bergen dabei immer das Risiko von optisch nicht ansprechenden Produktrückständen im haar ("flaking"), zudem wird bei der Steigerung des Haltegrades durch die Erhöhung des Polymeranteils üblicherweise ein Plateau erreicht, über das hinaus der haltegrad nur schwer durch weitere Polymererhöhung gesteigert werden kann.

Aufgabe der vorliegenden Erfindung war es daher, ein Mittel zur temporären Verformung keratinischer Fasern zur Verfügung zu stellen, das sich durch einen hohen bis höchsten Haltegrad auszeichnet und keine flaking-Probleme aufweist. Zudem sollten die Haltegrade mit moderaten Polymermengen erreicht werden können, und die Haltegrade sollten unmittelbar nach der Applikation erreicht werden, ohne daß es langer Trockenzeiten bedarf.

Es wurde nunmehr überraschenderweise gefunden, dass dies durch die erfindungsgemäße Polymerkombination erreicht werden kann.

Gegenstand der vorliegenden Erfindung sind in einer ersten Ausführungsform Mittel zur Behandlung keratinhaltiger Fasern, insbesondere menschlichem Haar, enthaltend in einem kosmetisch akzeptablen Träger-jeweils bezogen auf ihr Gewicht -
(a) 1 bis 20 Gew.-% mindestens eines vernetzten, amphiphilen, anionischen Polymers, umfassend mindestens eine Struktureinheit der Formel (I) und mindestens eine Struktureinheit der Formel (II), 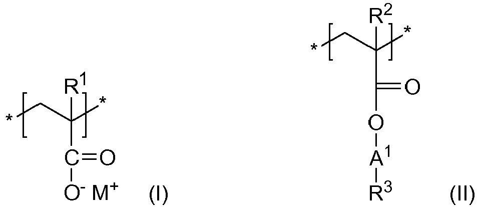 worin
   R¹ und R² stehen unabhängig voneinander für ein Wasserstoffatom oder eine Methylgruppe,
   R³ steht für eine (C₈ bis C₃₀)-Alkylgruppe,
   M⁺ steht für ein physiologisch verträgliches Kation und
   A¹ steht für eine Gruppe *-(CH₂CH₂O)ₓ-* worin x für eine ganze Zahl von 5 bis 35 steht, eine Gruppe *-(CH₂CHMeO)_{y}-* worin y für eine ganze Zahl von 5 bis 35 steht oder eine Gruppe
   *-(CH₂CH_{2O})ₓ(CH₂CHMeO)y-* worin die Summe x + y für eine ganze Zahl von 5 bis 35 steht und x und y größer als Null sind
   und
(b) 1 bis 20 Gew.-% mindestens eines unvernetzten, anionischen Polymers, umfassend mindestens eine Struktureinheit der Formel (III) und mindestens eine Struktureinheit der Formel (IV), 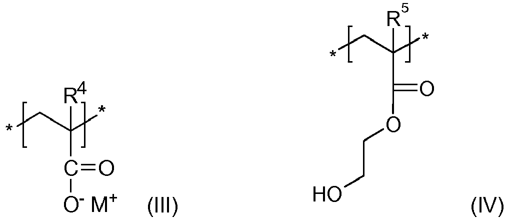 worin
   R⁴ und R⁵ stehen unabhängig voneinander für ein Wasserstoffatom oder eine Methylgruppe,
   M⁺ steht für ein physiologisch verträgliches Kation
(c) 1 bis 40 Gew.-% mindestens eines festigenden nichtionischen Polymers aus der Gruppe
   (c1) der nichtionischen Polymere mit mindestens einem Strukturelement der Formel (N5) 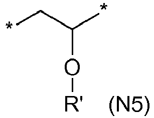 in der R' für ein Wasserstoffatom, eine Alkylgruppe oder eine Alkanoylgruppe, vorzugsweise für eine Acetylgruppe, steht,
   (c2) der Homopolymeren und nichtionischen Copolymeren des N-Vinylpyrrolidons,
   (c3) der nichtionischen Copolymeren des Isobutens.

Beispiele für erfindungsgemäße (C₁ bis C₄)-Alkylgruppen sind Methyl, Ethyl, Propyl, Isopropyl, Butyl, Sec-Butyl, Isobutyl, tert-Butyl. Beispiele für erfindungsgemäße (C₈ bis C₃₀)-Alkylgruppen sind Octyl (Capryl), Decyl (Caprinyl), Dodecyl (Lauryl), Tetradecyl (Myristyl), Hexadecyl (Cetyl), Octadecyl (Stearyl), Eicosyl (Arachyl), Docosyl (Behenyl).

Gemäß obiger Formeln und allen folgenden Formeln steht eine chemische Bindung, die mit dem Symbol * gekennzeichnet ist, für eine freie Valenz des entsprechenden Strukturfragments.

Insbesondere sind als physiologisch verträgliche Kationen M⁺ zur Kompensation der negativen Ladung der amphiphilen, anionischen Polymere Metallkationen der physiologisch verträglichen Metalle aus den Gruppen la, Ib, IIa, IIb, IIIb, VIa oder VIII des Periodensystems der Elemente, Ammoniumionen, sowie kationische organische Verbindungen mit quaterniertem Stickstoffatom geeignet. Letztere werden beispielsweise durch Protonierung primärer, sekundärer oder tertiärer organischer Amine mit einer Säure, oder durch permanente Quaternisierung besagter organischer Amine gebildet. Beispiele dieser kationischen organischen Ammoniumverbindungen sind 2-Ammonioethanol und 2-Trimethylammonioethanol.

Unter "vernetzt" bzw. "Vernetzung" ist im Sinne der Erfindung die Verknüpfung von Polymerketten miteinander durch kovalente chemische Bindung unter Bildung eines Netzwerkes zu verstehen. Diese kovalente Verknüpfung der Polymerketten darf mittels direkter kovalenter Bindung erfolgen oder durch ein die Polymerketten verbrückendes Molekülfragment vermittelt werden. Das Molekülfragment bindet an die durch das Molekülfragment verbrückten Polymerketten jeweils mittels kovalenter chemischer Bindung. Unter "unvernetzt" ist im Sinne der Erfindung zu verstehen, dass keine zuvor definierte "Vernetzung" vorliegt.

Unter "amphiphil" versteht der Fachmann im Allgemeinen die Tatsache, dass ein und dasselbe Molekül hydrophile Strukturelemente (beispielsweise solche der Formeln I bzw. (III)) und lipophile Strukturelemente (beispielsweise solche der Formeln (II) bzw. (IV)) umfasst.

Erfindungsgemäß bevorzugte Mittel sind dadurch gekennzeichnet, daß gemäß Formel (II) A¹ für eine Gruppe *-(CH₂CH₂O)c-* steht, worin x für eine ganze Zahl von 5 bis 35, insbesondere für eine ganze Zahl von 10 bis 24, steht.

Erfindungsgemäß weiter bevorzugte Mittel sind dadurch gekennzeichnet, daß gemäß Formel (II) R² bevorzugt für eine Methylgruppe steht.

Die Vernetzung der vernetzen, amphiphilen, anionischen Polymere (a) kann bevorzugt durch Verwendung mindestens eines vernetzenden Monomers bewerkstelligt werden. Dabei ist es wiederum bevorzugt die vernetzenden Monomere aus mindestens einer Verbindung der Gruppe zu wählen, die gebildet wird aus polyungesättigten aromatischen Monomeren (wie beispielsweise Divinylbenzol, Divinylnaphthalin, Trivinylbenzol), polyungesättigten alicyclischen Monomeren (wie beispielsweise 1,2,4-Trivinylcyclohexan), di-funktionellen Estern der Phthalsäure (wie beispielsweise Diallylphthalat), polyungesättigte aliphatische Monomere (wie beispielsweise Diene, Triene,Tetraene wie Isopren, 1,3-Butadien, 1,5-Hexadien, 1,5,9-Decatrien, 1,9-Decadien, 1,5-Heptadien), Polyalkenylether (wie beispielsweise Triallylpentaerythritol, Diallylpentaerythritol, Diallylsucrose, Octaallylsucrose, Trimethylolpropandiallylether), polyungesättigte Ester von Polyalkoholen oder Polysäuren (wie beispielsweise 1,6-Hexandioldi(meth)acrylat, Tetramethylentri(meth)acrylat, Allylacrylat, Diallylitaconat, Diallylfumarat, Diallylmaleat, Trimethylolpropantri(meth)acrylat, Trimethylolpropandi(meth)acrylat, Polyethyleneglycoldi(meth)acrylat), Alkylenebisacrylamide (wie beispielsweise Methylenbisacrylamid, Propylenbisacrylamid) Hydroxy- und Carboxyderivate des Methylenbisacrylamids (wie beispielsweise N,N'-Bismethylolmethylenbisacrylamid), Polyethyleneglycoldi(meth)acrylate (wie beispielsweise Ethyleneglycoldi(meth)acrylat, Diethyleneglycoldi(meth)acrylat, Triethyleneglycoldi(meth)acrylat), polyungesättigte Silane (wie beispielsweise Dimethyldivinylsilan, Methyltrivinylsilan, Allyldimethylvinylsilan, Diallyldimethylsilan, Tetravinylsilan), N-Methylolacrylamid; N-alkoxy(meth)acrylamid, wobei die Alkoxygruppe eine (C₁ bis C₁₈)-Alkoxygruppe ist, ungesättigte hydrolysierbare Silane (wie beispielsweise Triethoxyvinylsilan, Trisisopropoxyvinylsilan, 3-Triethoxysilylpropylmethacrylat), hydrolyierbare Silane (wie beispielsweise Ethyltriethoxysilan, Ethyltrimethoxysilan), Epoxy-substituierte hydrolysierbare Silane (wie beispielsweise 2-(3,4-Epoxycyclohexyl)ethyltriethoxysilan, 3-Glycidoxypropyltrimethyoxysilan) Polyisocyanate (wie beispielsweise 1,4-Diisocyanatobutan, 1,6-Diisocyanatohexan, 1,4-Phenylenediisocyanat, 4,4'-Oxybis(phenylisocyanat), ungesättigte Epoxide (wie beispielsweise Glycidylmethacrylate, Allylglycidylether), Polyepoxide (wie beispielsweise Diglycidylether, 1,2,5,6-Diepoxyhexan, Ethylenglycoldiglycidylether), ethoxylierte Polyole (wie beispielsweise Diole, Triole und Diphenole, jeweils ethoxyliert mit 2 bis 100 mol Ethyleneoxid pro Mol Hydroxylgruppen und terminiert mit einer polymerisierbaren ungesättigten Gruppe, wie beispielsweise Vinylether, Allylether, Acrylateester, Methacrylateester; Beispiele umfassen Bisphenol A ethoxyliertes di(meth)acrylat, Bisphenol F ethoxyliertes Di(meth)acrylat, ethoxyliertes Trimethylolpropantri(meth)acrylate, Acrylat- und Methacrylatester von Polyolen mit mindestens zwei Acrylatester- oder Methacrylatester-Funktionalitäten (wie beispielsweise Trimethylolpropantriacrylat (TMPTA), Trimethylolpropanethoxylated (15) triacrylat (TMPEO15TA), Trimethylolpropandimethacrylat, Triethyleneglycoldimethacrylat (TEGDMA), mit 30 Mol Ethylenoxid ethoxylliertes Bisphenol A-dimethacrylat (EOBDMA)).

Die vernetzten, amphiphilen, anionischen Polymere (a) sind in den erfindungsgemäßen Mitteln bevorzugt in einer Menge von 0,1 Gew.-% bis 5,0 Gew.-%, besonders bevorzugt von 0,2 Gew.-% bis 2 Gew.-%, ganz besonders bevorzugt von 0,2 Gew.-% bis 1,5 Gew.-% - jeweils bezogen auf das Gewicht des gesamten Mittels - enthalten.

Besonders bevorzugte erfindungsgemäße vernetzte amphiphile anionische Polymere (a) enthalten zusätzlich mindestens eine Struktureinheit der Formel (V) 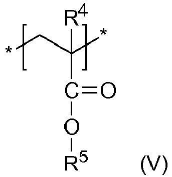 worin
R⁴ steht für ein Wasserstoffatom oder eine Methylgruppe,
R⁵ steht für eine (C₂ bis C₄)-Alkylgruppe, insbesondere für Ethyl.

Es ist erfindungsgemäß bevorzugt, als vernetztes amphiphiles, anionisches Polymer (a) mindestens ein Polymer auszuwählen, umfassend mindestens eine Struktureinheit der Formel (I) und mindestens eine Struktureinheit der Formel (II-a), 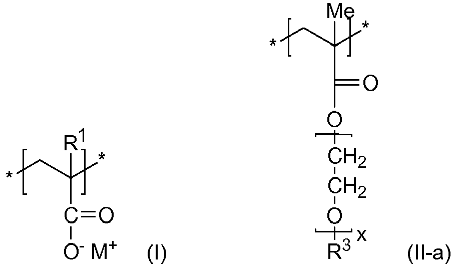 worin
R¹ steht für ein Wasserstoffatom oder eine Methylgruppe,
R³ steht für eine (C₈ bis C₃₀)-Alkylgruppe,
M⁺ steht für ein physiologisch verträgliches Kation und
x steht für eine ganze Zahl von 5 bis 35, insbesondere für eine ganze Zahl von 10 bis 24.

Darüberhinaus ist es besonders bevorzugt, die vernetzten, amphiphilen, anionischen Polymere (a) aus mindestens einem Polymer auszuwählen, umfassend mindestens eine Struktureinheit der Formel (I) und mindestens eine Struktureinheit der Formel (II-a) und mindestens eine Struktureinheit der Formel (V), 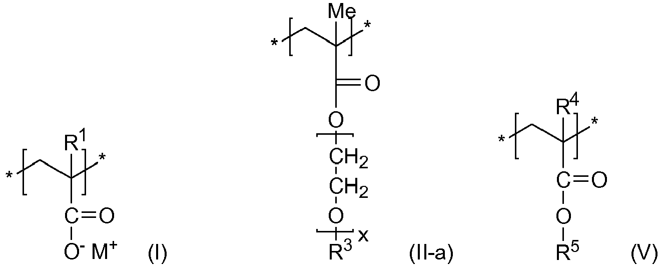 worin
R¹ und R⁴ stehen unabhängig voneinander für ein Wasserstoffatom oder eine Methylgruppe,
R³ steht für eine (C₈ bis C₃₀)-Alkylgruppe,
R⁵ steht für eine (C₂ bis C₄)-Alkylgruppe, insbesondere für Ethyl,
M⁺ steht für ein physiologisch verträgliches Kation und
x steht für eine ganze Zahl von 5 bis 35, insbesondere für eine ganze Zahl von 10 bis 24.

Ein ganz besonders bevorzugtes Polymer (a) ist ein vernetztes, amphiphiles, anionisches Polymer, das unter die INCI-Bezeichnung Acrylates / Steareth-20 Methacrylate Crosspolymer fällt. Es besitzt 20 Einheiten Ethylenoxid (x gemäß Formel (II-a) = 20) und ist mit Stearylalkohol verethert (R³ gemäß Formel (II-a) = Stearyl) Solche Polymere werden beispielsweise mit dem Handelsnamen Aculyn^{®} 88 von der Firma Rohm & Haas in Form einer 28 bis 30 Gew.-%-igen Dispersion in Wasser vertrieben.

Weiterhin enthält das erfindungsgemäße Mittel zu den zuvor definierten vernetzten amphiphilen, anionischen Polymeren zwingend mindestens ein zuvor definiertes unvernetztes, amphiphiles, anionisches Polymer (b).

Im Sinne der Erfindung bevorzugte Mittel enthalten die unvernetzten, amphiphilen, anionischen Polymere (b) in einer Menge von 0,1 Gew.-% bis 5,0 Gew.-%, besonders bevorzugt von 0,2 Gew.-% bis 5,0 Gew.-%, ganz besonders bevorzugt von 0,1 bis 2,0 Gew.%, jeweils bezogen auf das Gewicht des Mittels.

Die vernetzten, amphiphilen, anionischen Polymere (a) und die unvernetzten, amphiphilen, anionischen Polymere (b) werden erfindungsgemäß bevorzugt in einem Gewichtsverhältnis [Polymer (a) zu Polymer (b)] von 1 zu 5 bis 5 zu 1, insbesondere von 1 zu 2 bis 5 zu 1, ganz besonders bevorzugt von 1 zu 1.5 bis 2 zu 1, eingesetzt.

Gemäß Formel (IV) steht A² bevorzugt für eine Gruppe *-(CH₂CH₂O)ₓ-* worin x für eine ganze Zahl von 5 bis 35, insbesondere für eine ganze Zahl von 15 bis 30, steht.

Gemäß Formel (IV) steht R⁵ bevorzugt für ein Wasserstoffatom.

Erfindungsgemäß bevorzugte Mittel sind dadurch gekennzeichnet, daß das unvernetzte, anionische Polymer (b) zusätzlich mindestens eine Struktureinheit der Formel (V) enthält, 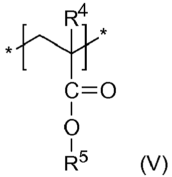 worin
R⁴ steht für ein Wasserstoffatom oder eine Methylgruppe,
R⁵ steht für eine (C₂ bis C₄)-Alkylgruppe, insbesondere für Methyl oder n-Butyl.

Besonders bevorzugte erfindungsgemäße unvernetzte amphiphile anionische Polymere (b) enthalten zusätzlich mindestens eine Struktureinheit der Formel (V) 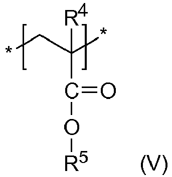 worin
R⁴ steht für ein Wasserstoffatom oder eine Methylgruppe,
R⁵ steht für eine (C₂ bis C₄)-Alkylgruppe, insbesondere für Ethyl.

Äußerst bevorzugte erfindungsgemäße Mittel sind dadurch gekennzeichnet, daß das unvernetzte, anionische Polymer (b) ein lineares Tetrapolymer aus Methacrylsäure, Hydroxyethylmethacrylat, Methylmethacrylat und Butylacrylat ist.

Zusätzlich zu den zwei vorstehend genannten Polymeren enthalten die erfindungsgemäßen Mittel als drittes Polymer 1 bis 40 Gew.-% mindestens eines festigenden nichtionischen Polymers aus der Gruppe
(c1) der nichtionischen Polymere mit mindestens einem Strukturelement der Formel (N5) 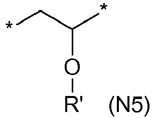 in der R' für ein Wasserstoffatom, eine Alkylgruppe oder eine Alkanoylgruppe, vorzugsweise für eine Acetylgruppe, steht,
(c2) der Homopolymeren und nichtionischen Copolymeren des N-Vinylpyrrolidons,
(c3) der nichtionischen Copolymeren des Isobutens.

Bevorzugt als zusätzliches filmbildendes und/oder festigendes Polymer geeignete nichtionische Polymere auf Basis ethylenisch ungesättigter Monomere, sind solche nichtionischen Polymere, welche mindestens eine der nachfolgenden Struktureinheiten enthalten 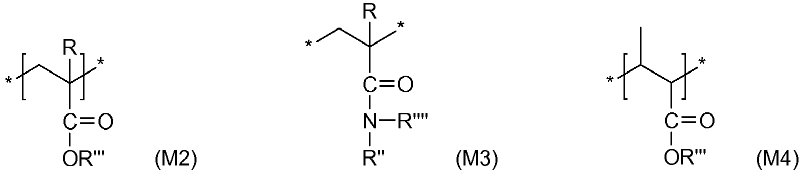 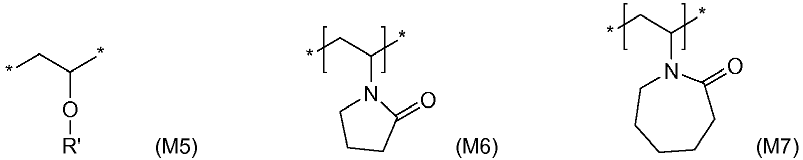 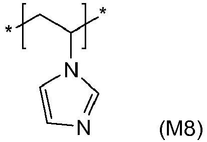 worin
- R: steht für ein Wasserstoffatom oder eine Methylgruppe,
- R': steht für ein Wasserstoffatom oder eine (C₁ bis C₄)-Acylgruppe,
- R" und R"": stehen unabhängig voneinander für eine (C₁ bis C₇)-Alkylgruppe oder ein Wasserstoffatom
- R"': steht für eine lineare oder verzweigte (C₁ bis C₄)-Alkylgruppe oder eine (C₂ bis C₄)-Hydroxyalkylgruppe.

Bevorzugte, nichtionische filmbildende und/oder nichtionische haarfestigende Polymere sind Homo-oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, wobei jeweils die Alkylgruppen dieser Monomere aus (C₁ bis C₃)-Alkylgruppen ausgewählt werden.

Für die erfindungsgemäßen Mittel besonders geeignete nichtionische Polymere auf Basis ethylenisch ungesättigter Monomere enthalten mindestens eine der nachfolgenden Struktureinheiten 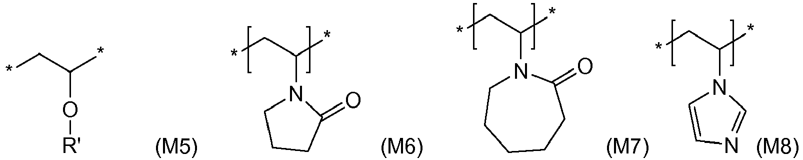 worin
- R': steht für ein Wasserstoffatom oder eine (C₁- bis C₃₀)-Acylgruppe, insbesondere für ein Wasserstoffatom oder eine Acetylgruppe.

Geeignet sind insbesondere Homopolymere des Vinylcaprolactams oder des Vinylpyrrolidons (wie beispielsweise Luviskol^{®} K 90 oder Luviskol^{®} K 85 der Firma BASF SE), Copolymerisate aus Vinylpyrrolidon und Vinylacetat (wie sie beispielsweise unter dem Warenzeichen Luviskol^{®} VA 37, Luviskol^{®} VA 55, Luviskol^{®} VA 64 und Luviskol^{®} VA 73 von der Firme BASF SE vertrieben werden), Terpolymere aus Vinylpyrrolidon, Vinylacetat und Vinylpropionat, Polyacrylamide (wie beispielsweise Akypomine^{®} P 191 von der Firma CHEM-Y), Polyvinylalkohole (die beispielsweise unter den Handelsbezeichnungen Elvanol^{®} von Du Pont oder Vinol^{®} 523/540 von der Firma Air Products vertrieben werden), Terpolymere aus Vinylpyrrolidon, Methacrylamid und Vinylimidazol (wie beispielsweise Luviset^{®} Clear der Firma BASF SE).

Neben den auf ethylenisch ungesättigten Monomeren basierenden nichtionischen Polymeren eignen sich weiterhin zur bevorzugten Ausführung der technischen Lehre nichtionische Cellulosederivate als filmbildende und/oder festigende Polymere, die bevorzugt ausgewählt werden aus Methylcellulose und insbesondere aus Celluloseether, wie Hydroxypropylcellulose (z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird), Hydroxyethylcellulose, wie sie beispielsweise unter den Warenzeichen Culminal^{®} und Benecel^{®} (AQUALON) und Natrosol^{®}-Typen (Hercules) vertrieben werden.

Zusammenfassend sind erfindungsgemäße Mittel bevorzugt, die als festigendes nichtionisches Polymer (c) mindestens ein Polymer aus der Gruppe
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
- Copolymere aus N-Vinylpyrrolidon mit N,N-Di(C₁ bis C₄)-Alkylamino-(C₂ bis C₄)-alkylacrylamid,
enthalten.

Unabhängig von dm/den jeweilig eingesetzten Polymer(en) werden die Polymere vorzugsweise innerhalb engerer Mengenbereiche eingesetzt. Durch die erfindungsgemäße Kombination sind höchste Haltegrade bereits durch geringe Polymermengen realisierbar. Hier sind erfindungsgemäße Mittel bevorzugt, die - bezogen auf ihr Gewicht -
- die vernetzten, amphiphilen, anionischen Polymere (a) in einer Menge von 1,5 bis 17,5 Gew.-%, vorzugsweise von 2 bis 15 Gew.-%, weiter bevorzugt von 3 bis 12,5 Gew.-% und insbesondere von 5 bis 10 Gew.-%,
- die unvernetzten, anionischen Polymere (b) in einer Menge von 1,5 bis 17,5 Gew.-%, vorzugsweise von 2 bis 15 Gew.-%, weiter bevorzugt von 2,5 bis 12,5 Gew.-% und insbesondere von 3 bis 10 Gew.-%,
- die festigenden nichtionischen Polymere (c) in einer Menge von 1,5 bis 35 Gew.-%, vorzugsweise von 2 bis 30 Gew.-%, weiter bevorzugt von 3 bis 25 Gew.-% und insbesondere von 5 bis 20 Gew.-%,
enthalten.

Es hat sich für die Erreichung höchster Haltegrade und für die Minimierung von flaking-Erscheinungen als ganz besonders bevorzugt herausgestellt, die Polymere (a) und (b) innerhalb bestimmter Verhältnisse zueinander einzusetzen. Hier sind erfindungsgemäße Mittel bevorzugt, bei denen die vernetzten, amphiphilen, anionischen Polymere (a) und die unvernetzten anionischen Polymere (b) in einem Gewichtsverhältnis von 1 zu 5 bis 5 zu 1, insbesondere von 1 zu 2 bis 5 zu 1, besonders bevorzugt von 1 zu 1.5 bis 2 zu 1, eingesetzt werden.

Zusätzlich zu den Polymeren können die erfindungsgemäßen Mittel mindestens ein Wachses mit einem Schmelzpunkt in einem Bereich von 40°C bis 90°C enthalten.

Generell sind Wachse von fester bis brüchig harter Konsistenz, grob bis feinkristallin, durchscheinend bis opak, jedoch nicht glasartig, und schmelzen oberhalb von 40°C ohne Zersetzung. Sie sind schon wenig oberhalb des Schmelzpunktes niedrigviskos und zeigen eine stark temperaturabhängige Konsistenz und Löslichkeit. Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse, insbesondere Polyethylenwachse, und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren.

Eine besonders bevorzugte Wachskomponente ist ausgewählt aus mindestens einem Ester aus einem gesättigten, einwertigen C₁₆-C₅₀-Alkohol und einer gesättigten C₈-C₃₆-Monocarbonsäure. Erfindungsgemäß zählen hierzu auch Lactide, die cyclischen Doppelester von alpha-Hydroxycarbonsäuren der entsprechenden Kettenlänge. Ester aus Fettsäuren und langkettigen Alkoholen haben sich für die erfindungsgemäß bevorzugten Zusammensetzungen als besonders vorteilhaft erwiesen. Die Ester setzen sich aus gesättigten, verzweigten oder unverzweigten Monocarbonsäuren und gesättigten, verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten, verzweigten oder unverzweigten Alkoholen sind erfindungsgemäß einsetzbar, sofern die Wachskomponente einen Schmelzpunkt > 50°C hat. Besonders bevorzugt ist, die Wachskomponenten zu wählen aus der Gruppe der Ester aus gesättigten, verzweigten oder unverzweigten Alkancarbonsäuren einer Kettenlänge von 12 bis 24 C-Atomen und den gesättigten, verzweigten oder unverzweigten Alkoholen einer Kettenlänge von 16 bis 50 C-Atomen, die einen Schmelzpunkt > 50°C haben.

Insbesondere können als Wachskomponente C₁₆₋₃₆-Alkylstearate und C₁₈₋₃₈-Alkylhydroxystearoylstearate, C₂₀₋₄₀-Alkylerucate sowie Cetearylbehenat bevorzugt sein. Das Wachs oder die Wachskomponenten weisen einen Schmelzpunkt > 50°C, bevorzugt > 60°C, auf. Eine besonders bevorzugte Ausführungsform der Erfindung enthält als Wachskomponente ein C₂₀-C₄₀-Alkylstearat. Dieser Ester ist unter den Namen Kesterwachs^{®} K82H oder Kesterwachs^{®} K80H bekannt und wird von Koster Keunen Inc. vertrieben. Es handelt sich um die synthetische Nachahmung der Monoesterfraktion des Bienenwachses und zeichnet sich durch seine Härte, seine Ölgelierfähigkeit und seine breite Kompatibilität mit Lipidkomponenten aus. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs^{®} K62 bekannt und wird von Koster Keunen Inc. vertrieben.

Weitere bevorzugte Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2-6 Kohlenstoffatomen, solange sie einen Schmelzpunkt oberhalb von 50°C aufweisen, beispielsweise bevorzugt C₁₈-C₃₈ Acid Triglyceride (Syncrowax^{®} HGL-C). Erfindungsgemäß ist als Wachskomponente hydriertes Rizinusöl, erhältlich z. B. als Handelsprodukt Cutina^{®} HR, besonders bevorzugt.

Weitere bevorzugte Lipid- oder Wachskomponenten mit einem Schmelzpunkt > 50°C sind die gesättigten linearen C₁₄-C₃₆-Carbonsäuren, insbesondere Myristinsäure, Palmitinsäure, Stearinsäure und Behensäure sowie Mischungen dieser Verbindungen, z. B. Syncrowax^{®} AW IC (C₁₈-C₃₆-Fettsäuren) oder Cutina^{®} FS 45 (Palmitin- und Stearinsäure).

Weitere bevorzugte Lipid- oder Wachskomponenten mit einem Schmelzpunkt im Bereich von 30-150°C sind lineare, gesättigte C₈₋₃₀-Fettsäuren. Bevorzugt sind lineare, gesättigte C₁₀₋₂₂-Fettsäuren. Bevorzugte Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Elaeostearinsäure, Arachidonsäure, Gadoleinsäure, Behensäure und Erucasäure sowie deren technische Mischungen. Besonders bevorzugt ist der Einsatz von Stearinsäure. Die eingesetzten Fettsäuren können eine oder mehrere Hydroxygruppen tragen. Bevorzugte Beispiele hierfür sind die &agr;-Hydroxy-C₈-C₁₈-Carbonsäuren sowie 12-Hydroxystearinsäure.

Weitere bevorzugte Lipid- oder Wachskomponenten mit einem Schmelzpunkt im Bereich von 30 - 150°C sind Fettalkohole. Als Fettalkohole können eingesetzt werden gesättigte, unverzweigte Fettalkohole mit 6-30, bevorzugt 10-22 und ganz besonders bevorzugt 12-22 Kohlenstoffatomen. Einsetzbar im Sinne der Erfindung sind z. B. Decanol, Octanol, Erucaalkohol, Ricinolalkohol, 12-Hydroxystearylalkohol, Stearylalkohol, Cetylalkohol, Laurylalkohol, Myristylalkohol, Arachidylalkohol, Caprylalkohol, Caprinalkohol und Behenylalkohol.

Bevorzugte erfindungsgemäße Zusammensetzungen sind dadurch gekennzeichnet, dass die Wachskomponente ausgewählt ist aus Estern aus einem gesättigten, einwertigen C₁₆-C₆₀-Alkanol und einer gesättigten C₆-C₃₆-Monocarbonsäure, insbesondere Cetylbehenat, Stearylbehenat und C₂₀-C₄₀-Alkylstearat, Glycerintriestern von gesättigten linearen C₁₂-C₃₀-Carbonsäuren, die hydroxyliert sein können, Candelillawachs, Carnaubawachs, Bienenwachs, gesättigten linearen C₁₄-C₃₆-Carbonsäuren sowie Mischungen der vorgenannten Substanzen. Besonders bevorzugte Wachskomponenten-Mischungen sind ausgewählt aus Mischungen von Cetylbehenat, Stearylbehenat, gehärtetem Rizinusöl, Palmitinsäure und Stearinsäure. Weitere besonders bevorzugte Wachskomponenten-Mischungen sind ausgewählt aus Mischungen von C₂₀-C₄₀-Alkylstearat, gehärtetem Rizinusöl, Palmitinsäure und Stearinsäure.

Besonders bevorzugte erfindungsgemäße Zusammensetzungen sind dadurch gekennzeichnet, dass die Wachskomponente ausgewählt ist aus Mischungen von Estern aus einem gesättigten, einwertigen C₁₆-C₆₀-Alkanol und einer gesättigten C₈-C₃₆-Monocarbonsäure, insbesondere C₂₀-C₄₀-Alkylstearat, Glycerintriestern von gesättigten linearen C₁₂-C₃₀-Carbonsäuren, die hydroxyliert sein können, insbesondere hydriertem Rizinusöl, und gesättigten linearen C₁₄-C₃₆-Carbonsäuren, insbesondere Palmitinsäure und Stearinsäure.

Die erfindungsgemäßen Mittel enthalten die Inhaltsstoffe in einem kosmetisch akzeptablen Träger. Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol, Butylenglycol und Propylenglykol in einer Menge bis 30 Gew.-% bezogen auf das gesamte Mittel.

Ganz besonders bevorzugte erfindungsgemäße Mittel sind hoch wasserhaltig. Es hat sich gezeigt, daß die Einstellung von Glanz, Remodulierbarkeit und Haltegrad bei den erfindungsgemäßen Zusammensetzungen besonders gut gelingt, wenn diese ho0he Anteile an Wasser enthalten. Besonders bevorzugte erfinddungsgemäße Mittel sind daher dadurch gekennzeichnet, daß sie bezogen auf ihr Gewicht 40 bis 95 Gew.-%, vorzugsweise 45 bis 92,5 Gew.-%, weiter bevorzugt 50 bis 90 Gew.-%, noch weiter bevorzugt 55 bis 87,5 Gew.-% und insbesondere 60 bis 85 Gew.-% Wasser enthalten.

Die Applizierbarkeit der erfindungsgemäßen Zusammensetzungen läßt sich durch den Einsatz geringer Mengen eines oder mehrerer mehrwertiger Alkohole weiter steigern. Bevorzugte erfindungsgemäße Mittel enthalten bezogen auf ihr Gewicht 0,25 bis 5 Gew.-%, vorzugsweise 0,5 bis 4 Gew.-%, weiter bevorzugt 0,75 bis 3 Gew.-% und insbesondere 1 bis 2,5 Gew.-% mindestens eines mehrwertigen Alkohols aus der Gruppe Glycerin und/oder Propandiol-1,2.

Die Mittel weisen bevorzugt einen pH-Wert von 2 bis 11 auf. Besonders bevorzugt ist der pH-Bereich zwischen 4 und 9. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise den jeweiligen kosmetischen Mitteln zugesetzt werden.

Als geeignete Hilfs- und Zusatzstoffe sind insbesondere Pflegestoffe zu nennen. Diese finden sowohl bei Haut- als auch Haarbehandlungsmitteln Anwendung und können bei geeigneter Wahl des Pflegestoffs beispielsweise in Cremes, Shampoos, Haarspülungen, Haarkuren, Gele, Pump- und Aerosolsprays und Schaumprodukte eingearbeitet werden.

Als Pflegestoff kann ein erfindungsgemäßes Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben. Besonders interessant ist der Einsatz von Seiden-Proteinhydrolysaten. Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff einer anderen Verbindungsklasse sind weiterhin kationische Tenside geeignet. Erfindungsgemäß bevorzugt sind kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Als Pflegestoff eignen sich ebenfalls pflegende Polymere. Es sei an dieser Stelle darauf hingewiesen, dass einige pflegende Polymere auch filmbildende und/oder festigende Eigenschaften aufweisen, und daher auch bei der Aufzählung geeigneter filmbildender und/oder festigender Polymere genannt sein können.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I),

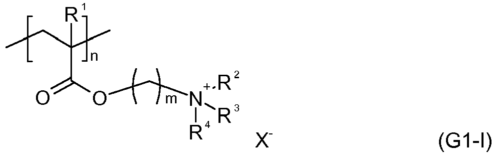

in der R¹= -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt: R¹ steht für eine Methylgruppe, R², R³ und R⁴ stehen für Methylgruppen, m hat den Wert 2.

Als physiologisch verträgliche Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silikon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®} 550 (Dimethyldiallylammoniumchlorid-Acrylamid-Copolymer) im Handel erhältlichen Produkte sind Beispiele für solche kationischen Polymere,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Die erfindungsgemäßen Mittel enthalten die pflegenden, kationischen und/oder amphoteren Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Weiter bevorzugte erfindungsgemäße Mittel sind dadurch gekennzeichnet, daß sie zusätzlich Pflegestoff(e) - bezogen auf ihr Gewicht - in Mengen von 0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% enthalten, wobei bevorzugte Pflegstoff(e) ausgewählt sind aus der Gruppe L-Carnitin und/oder seiner Salze; Panthenol und/oder Panthothensäure; der 2-Furanone und/oder deren Derivate (insbesondere Pantolacton); Taurin und/oder seiner Salze; Niacinamid; Ubichinon; Ectoin; Allantoin.

L-Carnitin (IUPAC-Name(R)-(3-Carboxy-2-hydroxypropyl)- *N,N,N-*trimethylammoniumhydroxid), ist eine natürlich vorkommende, vitaminähnliche Substanz. Erfindungsgemäß bevorzugte L-Carnitinderivate sind insbesondere ausgewählt aus Acetyl-L-Carnitin, L-Carnitin-Fumarat, L-Carnitin-Citrat, Lauroyl-L-Carnitin und besonderes bevorzugt L-Carnitin-Tartrat. Die genannten L-Carnitin-Verbindungen sind beispielsweise von der Firma Lonza GmbH (Wuppertal, Deutschland) erhältlich. Erfindungsgemäße bevorzugte Mittel sind dadurch gekennzeichnet, daß sie - bezogen auf ihr Gewicht -0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Panthenol (IUPAC-Name: (+)-(R)-2,4-Dihydroxy-N-(3-hydroxypropyl)-3,3-dimethylbutyramid) wird im Körper zu Pantothensäure umgewandelt. Pantothensäure ist ein Vitamin aus der Gruppe der B-Vitamine (Vitamin B5). Erfindungsgemäße bevorzugte Mittel sind dadurch gekennzeichnet, daß sie - bezogen auf sein Gewicht -0,01 bis 5 Gew.-%, vorzugsweise 0,05 bis 2,5 Gew.-%, besonders bevorzugt 0,1 bis 1,5 Gew.-% und insbesondere 0,25 bis 1 Gew.-% Panthenol ((±)-2,4-Dihydroxy-N-(3-hydroxypropyl)-3,3-dimethyl-butyramid) enthalten.

Ein weiterer, bevorzugter einsetzbarer Pflege-Enhancer, der aktivierende Eigenschaften besitzt, ist das Taurin. Erfindungsgemäß bevorzugte Mittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflege-Enhancerm in den erfindungsgemäßen Mitteln sind Vitamine, Provitamine oder Vitaminvorstufen. Diese werden nachfolgend beschrieben: Zusammenfassend sind erfindungsgemäße Mittel bevorzugt, die - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel -2,4-Dihydroxy-N-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, daß bestimmte Chinone eine besondere Eignung als Pflege-Enhancer besitzen. Besonders bevorzugte erfindungsgemäße Mittel sind dadurch gekennzeichnet, daß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 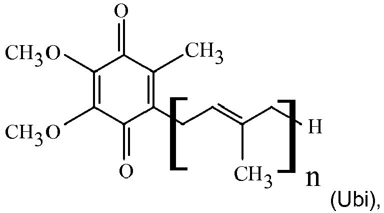 in der n für die Werte = 6, 7, 8, 9 oder 10, besonders bevorzugt für 10 (Coenzym Q10) steht.

Als weiteren Pflege-Enhacer können die erfindungsgemäßen Mittel Ectoin enthalten. Ectoin ((4S)-2-Methyl-1,4,5,6-Tetrahydropyrimidin-4-Carbonsäure) ist ein zur Gruppe der kompatiblen Solute gehörender Naturstoff.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Haarbehandlungsmittel dadurch gekennzeichnet, daß sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus
- Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose
- Disacchariden, insbesondere Saccharose und/oder Maltose und/oder Lactose und/oder Trehalose und/oder Cellobiose und/oder Gentiobiose und/oder Isomaltose.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel. Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

In Abhängigkeit von der Art des erfindungsgemäßen Mittels kann es erforderlich sein, dass diese weiterhin mindestens ein Tensid enthalten. Dies gilt insbesondere für Hautreinigungsmittel und Shampoos. Aber auch andere Mittel, wie beispielsweise Haarspülungen, Haarkuren und bestimmte Stylingmittel, insbesondere Stylingschäume, können Tenside enthalten.

Beispielsweise können kationische Tenside eingesetzt werden, wie sie bereits oben als geeignete Pflegestoffe beschrieben sind. Bezüglich der bevorzugten kationischen Tenside und der eingesetzten Mengen gelten obige Ausführungen entsprechend.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)_{X}-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)_{X}-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
   Alkyl- und/oder Alkenyletherphosphate
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine -COO⁽⁻⁾ - oder-SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈- Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR₂)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Der Alkyl- bzw. Alkenylrest R⁴ kann sich von primären Alkoholen mit 4 bis 11, vorzugsweise 8 bis 10 Kohlenstoffatomen ableiten. Bevorzugt sind Alkyloligoglucoside auf Basis von gehärtetem C_{12/14}-Kokosalkohol mit einem DP von 1 bis 3.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten. Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, eingesetzt. Dabei hängt die eingesetzte Menge wesentlich davon ab, welchen Zweck das erfindungsgemäße Mittel erfüllt. Handelt es sich um ein Shampoo oder ein anderes reinigendes Mittel, sind auch Tensidmengen über 45 Gew.-% üblich.

Die Formulierung der erfindungsgemäßen Mittel kann in allen für kosmetische Mittel üblichen Formen erfolgen, beispielsweise in Form von Lösungen, die als Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt. Da die erfindungsgemäß eingesetzte Polymerkombination jedoch selbstverdickende Eigenschaften aufweist, ist die Zugabe weiterer Strukturanten und/oder verdickender Polymere nicht zwingend erforderlich. Vorzugsweise enthalten die erfindungsgemäßen Mittel keine weiteren Strukturanten und/oder verdickender Polymere.

Sofern es sich bei den erfindungsgemäßen Mitteln um ein Aerosolprodukt handelt, enthält dieses zwingend ein Treibmittel. Erfindungsgemäß geeignete Treibmittel sind beispielsweise N₂O, Dimethylether, CO₂, Luft und Alkane mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und deren Mischungen. Bevorzugt werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe. Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Ein zweiter Gegenstand der Erfindung ist daher die Verwendung eines erfindungsgemäßen Mittels zur temporären Verformung von Haaren und/oder zur Haarpflege.

Ein dritter Gegenstand der Erfindung ist ein Verfahren zur Behandlung keratinhaltiger Fasern, insbesondere menschlicher Haare, worin ein erfindungsgemäßes Mittel auf die keratinhaltigen Fasern appliziert wird.

Für die erfindungsgemäße Verwendung und das erfindungsgemäße Verfahren gilt mutatis mutandis das zu den erfindungsgemäßen Mitteln Gesagte.

### Beispiele:

Die folgenden Mengenangaben verstehen sich - soweit nichts anderes vermerkt ist - in Gewichtsprozent.

Es wurden die erfindungsgemäßen Stylingmittel E1 bis E4 gemäß folgender Tabelle 1 hergestellt.

**Tabelle1**

| **Rohstoff (INCI-Bezeichnung)** | **E1** | **E2** | **E3** | **E4** |
|---|---|---|---|---|
| Aculyn^{®} 88¹ | 7,0 | 7,5 | 8,0 | 8,5 |
| Polymer (b)² | 5,0 | 5,5 | 6,0 | 7,0 |
| Polyvinylpyrrolidon | 5,0 | 5,0 | 5,0 | 5,0 |
| 2-Amino-2methyl-1-Propanol | 1,0 | 1,25 | 1,5 | 1,75 |
| Glycerin | 1,0 | 1,0 | 1,0 | 1,0 |
| Propandiol-1,2 | 1,0 | 1,0 | 1,0 | 1,0 |
| Phenoxyethanol | 0,5 | 0,5 | 0,5 | 0,5 |
| Farbstoff | 0,002 | 0,002 | 0,002 | 0,002 |
| Parfüm | 0,10 | 0,10 | 0,10 | 0,20 |
| Ethanol 96 %, vergällt | - | - | - | 10,70 |
| Wasser, entsalzt | ad 100 | ad 100 | ad 100 | ad 100 |

| | | | | |
|---|---|---|---|---|
| ¹ Copolymer aus (Meth)acrylsäure, (Meth)acrylsäureester und Steareth-20-Methacrylsäureester (28-30 Gew.-% Festkörper in Wasser; INCI-Bezeichnung: Acrylates/Steareth-20 Methacrylate Crosspolymer) (Rohm und Haas), ² lineares Tetrapolymer aus Methacrylsäure, Hydroxyethylmethacrylat, Methylmethacrylat und Butylacrylat | | | | |

Die Mittel wurden durch übliches Vermischen der in der Tabelle genannten Rohstoffe hergestellt und durch Verreiben auf den Handflächen und nachfolgende Applikation auf trockenes oder feuchtes Haar zur Frisurengestaltung eingesetzt.

Die Haare wiesen einen sanften Glanz, extremen und schnellen Frisurenhalt und kein flaking auf.

## Patentansprüche

1. Mittel zur Behandlung keratinhaltiger Fasern, insbesondere menschlichem Haar, enthaltend in einem kosmetisch akzeptablen Träger-jeweils bezogen auf sein Gewicht -
(a) 1 bis 20 Gew.-% mindestens eines vernetzten, amphiphilen, anionischen Polymers, umfassend mindestens eine Struktureinheit der Formel (I) und mindestens eine Struktureinheit der Formel (II), 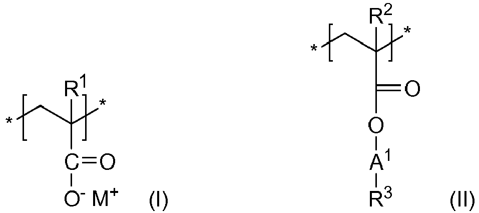 worin
R¹ und R² stehen unabhängig voneinander für ein Wasserstoffatom oder eine Methylgruppe,
R³ steht für eine (C₈ bis C₃₀)-Alkylgruppe,
M⁺ steht für ein physiologisch verträgliches Kation und
A¹ steht für eine Gruppe *-(CH₂CH₂O)ₓ-* worin x für eine ganze Zahl von 5 bis 35 steht, eine Gruppe *-(CH₂CHMeO)_{y}-* worin y für eine ganze Zahl von 5 bis 35 steht oder eine Gruppe
*-(CH₂CH₂O)ₓ-(CH₂CHMeO)_{y}-* worin die Summe x + y für eine ganze Zahl von 5 bis 35 steht und x und y größer als Null sind
und
(b) 1 bis 20 Gew.-% mindestens eines unvernetzten, anionischen Polymers, umfassend mindestens eine Struktureinheit der Formel (III) und mindestens eine Struktureinheit der Formel (IV). 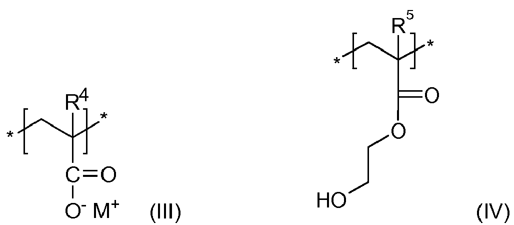 worin
R⁴ und R⁵ stehen unabhängig voneinander für ein Wasserstoffatom oder eine Methylgruppe,
M⁺ steht für ein physiologisch verträgliches Kation
(c) 1 bis 40 Gew.-% mindestens eines festigenden nichtionischen Polymers aus der Gruppe
(c1) der nichtionischen Polymere mit mindestens einem Strukturelement der Formel (N5) 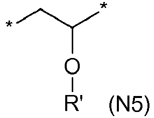 in der R' für ein Wasserstoffatom, eine Alkylgruppe oder eine Alkanoylgruppe, vorzugsweise für eine Acetylgruppe, steht,
(c2) der Homopolymeren und nichtionischen Copolymeren des N-Vinylpyrrolidons,
(c3) der nichtionischen Copolymeren des Isobutens.

2. Mittel nach Anspruch 1, **dadurch gekennzeichnet, daß** Gemäß Formel (II) A¹ für eine Gruppe *-(CH₂CH₂O)ₓ-* steht, worin x für eine ganze Zahl von 5 bis 35, insbesondere für eine ganze Zahl von 10 bis 24, steht.

3. Mittel nach einem der Ansprüche 1 oder 2, **dadurch gekennzeichnet, daß** gemäß Formel (II) R² bevorzugt für eine Methylgruppe steht.

4. Mittel nach einem der Ansprüche 1 bis 3, **dadurch gekennzeichnet, daß** das unvernetzte, anionische Polymer (b) zusätzlich mindestens eine Struktureinheit der Formel (V) enthält, 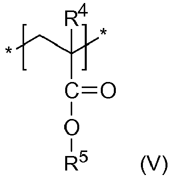 worin
R⁴ steht für ein Wasserstoffatom oder eine Methylgruppe,
R⁵ steht für eine (C₂ bis C₄)-Alkylgruppe, insbesondere für Methyl oder n-Butyl.

5. Mittel nach einem der Ansprüche 1 bis 4, **dadurch gekennzeichnet, daß** das unvernetzte, anionische Polymer (b) ein lineares Tetrapolymer aus Methacrylsäure, Hydroxyethylmethacrylat, Methylmethacrylat und Butylacrylat ist.

6. Mittel nach einem der Ansprüche 1 bis 5, **dadurch gekennzeichnet, daß** es als festigendes nichtionisches Polymer (c) mindestens ein Polymer aus der Gruppe
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
- Copolymere aus N-Vinylpyrrolidon mit N,N-Di(C₁ bis C₄)-Alkylamino-(C₂ bis C₄)-alkylacrylamid,
enthält.

7. Mittel nach einem der Ansprüche 1 bis 6, **dadurch gekennzeichnet, daß** es - bezogen auf sein Gewicht -
- die vernetzten, amphiphilen, anionischen Polymere (a) in einer Menge von 1,5 bis 17,5 Gew.-%, vorzugsweise von 2 bis 15 Gew.-%, weiter bevorzugt von 3 bis 12,5 Gew.-% und insbesondere von 5 bis 10 Gew.-%,
- die unvernetzten, anionischen Polymere (b) in einer Menge von 1,5 bis 17,5 Gew.-%, vorzugsweise von 2 bis 15 Gew.-%, weiter bevorzugt von 2,5 bis 12,5 Gew.-% und insbesondere von 3 bis 10 Gew.-%,
- die festigenden nichtionischen Polymere (c) in einer Menge von 1,5 bis 35 Gew.-%, vorzugsweise von 2 bis 30 Gew.-%, weiter bevorzugt von 3 bis 25 Gew.-% und insbesondere von 5 bis 20 Gew.-%,
enthält.

8. Mittel nach einem der Ansprüche 1 bis 7, **dadurch gekennzeichnet, daß** es die unvernetzten, amphiphilen, anionischen Polymere (b) in einer Menge von 0,1 Gew.-% bis 5,0 Gew.-%, bevorzugt von 0,2 Gew.-% bis 5,0 Gew.-%, besonders bevorzugt von 0,1 bis 2,0 Gew.%, jeweils bezogen auf das Gewicht des Mittels, enthält.

9. Mittel nach einem der Ansprüche 1 bis 8, **dadurch gekennzeichnet, dass** die vernetzten, amphiphilen, anionischen Polymere (a) und die unvernetzten anionischen Polymere (b) in einem Gewichtsverhältnis von 1 zu 5 bis 5 zu 1, insbesondere von 1 zu 2 bis 5 zu 1, besonders bevorzugt von 1 zu 1.5 bis 2 zu 1, eingesetzt werden.

10. Verwendung eines Mittels gemäß wenigstens eines der Ansprüche 1 bis 9 zur temporären Verformung von Haaren und/oder zur Haarpflege.

11. Verfahren zur Behandlung keratinhaltiger Fasern, insbesondere menschlicher Haare, worin ein Mittel gemäß wenigstens eines der Ansprüche 1 bis 9 auf die keratinhaltigen Fasern appliziert wird.

## Claims

1. An agent for treating keratin-containing fibers, in particular human hair, containing
(a) from 1 to 20 wt.% of at least one crosslinked, amphiphilic, anionic polymer comprising at least one structural unit of the formula (I) and at least one structural unit of the formula (II), 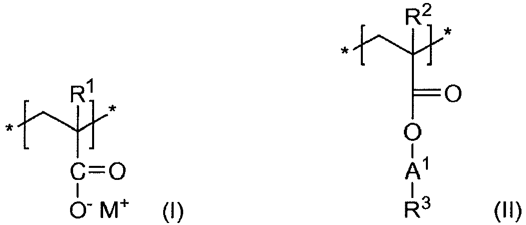 in which
R¹ and R² represent, independently of one another, a hydrogen atom or a methyl group,
R³ represents a (C₈ to C₃₀) alkyl group,
M⁺ represents a physiologically acceptable cation, and
A¹ represents a group *-(CH₂CH₂O)ₓ-*, in which x represents an integer from 5 to 35, a group *-(CH₂CHMeO)_{y}-*, in which y represents an integer from 5 to 35, or a group *-(CH₂CH₂O)ₓ-(CH₂CHMeO)y-*, in which the sum of x + y represents an integer from 5 to 35 and x and y are greater than zero,
and
(b) from 1 to 20 wt.% of at least one non-crosslinked, anionic polymer comprising at least one structural unit of the formula (III) and at least one structural unit of the formula (IV), 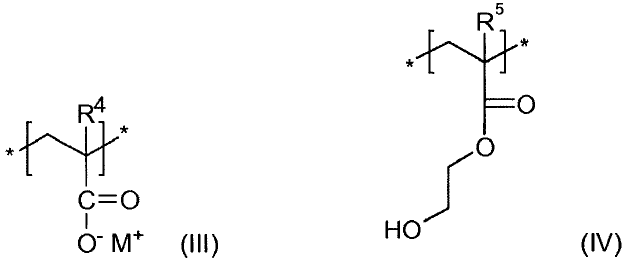 in which
R⁴ and R⁵ represent, independently of one another, a hydrogen atom or a methyl group, and
M⁺ represents a physiologically acceptable cation, and
(c) from 1 to 40 wt.% of at least one setting, non-ionic polymer from the group
(c1) of non-ionic polymers having at least one structural element of the formula (N5) 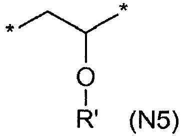 in which R' represents a hydrogen atom, an alkyl group or an alkanoyl group, preferably an acetyl group,
(c2) of homopolymers and non-ionic copolymers of N-vinylpyrrolidone, or
(c3) of non-ionic copolymers of isobutene,
in a cosmetically acceptable carrier, based on the weight of said agent in each case.

2. The agent according to claim 1, **characterized in that**, according to formula (II), A¹ represents a group *-(CH₂CH₂O)ₓ-*, in which x represents an integer from 5 to 35, in particular an integer from 10 to 24.

3. The agent according to one of claims 1 or 2, **characterized in that**, according to formula (II), R² preferably represents a methyl group.

4. The agent according to one of claims 1 to 3, **characterized in that** the non-crosslinked, anionic polymer (b) also contains at least one structural unit of the formula (V), 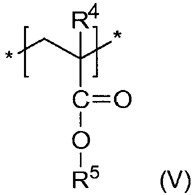 in which
R⁴ represents a hydrogen atom or a methyl group, and
R⁵ represents a (C₂ to C₄) alkyl group, in particular methyl or n-butyl.

5. The agent according to one of claims 1 to 4, **characterized in that** the non-crosslinked, anionic polymer (b) is a linear tetrapolymer made of methacrylic acid, hydroxyethyl methacrylate, methyl methacrylate and butyl acrylate.

6. The agent according to one of claims 1 to 5, **characterized in that** said agent contains at least one polymer from the group
- polyvinylpyrrolidone,
- copolymers of N-vinylpyrrolidone and vinyl esters of carboxylic acids having from 2 to 18 carbon atoms, in particular of N-vinylpyrrolidone and vinyl acetate,
- copolymers of N-vinylpyrrolidone and N-vinylimidazole and methacrylamide,
- copolymers of N-vinylpyrrolidone and N-vinylimidazole and acrylamide,
- copolymers of N-vinylpyrrolidone having N,N-Di(C₁ to C₄)-alkylamino-(C₂ to C₄)-alkylacrylamide, and
- copolymers of N-vinylpyrrolidone having N,N-Di(C₁ to C₄)-alkylamino-(C₂ to C₄)-alkylacrylamide
as the setting, non-ionic polymer (c).

7. The agent according to one of claims 1 to 6, **characterized in that** said agent contains, based on its weight,
- the crosslinked, amphiphilic, anionic polymers (a) in a quantity of from 1.5 to 17.5 wt.%, preferably from 2 to 15 wt.%, more preferably from 3 to 12.5 wt.% and in particular from 5 to 10 wt.%,
- the non-crosslinked, anionic polymers (b) in a quantity of from 1.5 to 17.5 wt.%, preferably from 2 to 15 wt.%, more preferably from 2.5 to 12.5 wt.% and in particular from 3 to 10 wt.%,
- the setting, non-ionic polymers (c) in a quantity of from 1.5 to 35 wt.%, preferably from 2 to 30 wt.%, more preferably from 3 to 25 wt.% and in particular from 5 to 20 wt.%.

8. The agent according to one of claims 1 to 7, **characterized in that** said agent contains the non-crosslinked, amphiphilic, anionic polymers (b) in a quantity of from 0.1 wt.% to 5.0 wt.%, preferably from 0.2 wt.% to 5.0 wt.%, particularly preferably from 0.1 to 2.0 wt.%, based on the weight of the agent in each case.

9. The agent according to one of claims 1 to 8, **characterized in that** the crosslinked, amphiphilic, anionic polymers (a) and the non-crosslinked, anionic polymers (b) are used in a weight ratio of from 1:5 to 5:1, in particular from 1:2 to 5:1, particularly preferably from 1:1.5 to 2:1.

10. The use of an agent according to at least one of claims 1 to 9 for temporarily styling hair and/or for hair care.

11. A method for treating keratin-containing fibers, in particular human hair, wherein an agent according to at least one of claims 1 to 9 is applied to the keratin-containing fibers.

## Revendications

1. Agent de traitement de fibres kératiniques, en particulier du cheveu humain, contenant dans un support cosmétiquement acceptable - par rapport à son poids respectivement -
(a) 1 à 20 % en poids d'au moins un polymère anionique, amphipathique, réticulé, comprenant au moins un motif structural de formule (I) et au moins un motif structural de formule (II), 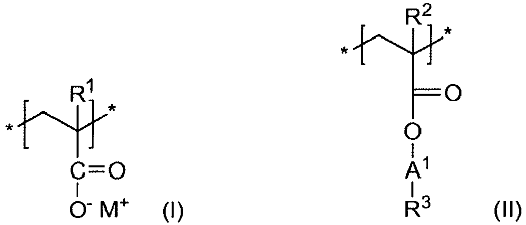 où
R¹ et R² représentent indépendamment l'un de l'autre un atome d'hydrogène ou un groupe méthyle,
R³ représente un groupe alkyle (en C₈ à C₃₀),
M⁺ représente un cation physiologiquement acceptable et
A¹ représente un groupe *-(CH₂CH₂O)ₓ-* où x représente un nombre entier de 5 à 35,
un groupe *-(CH₂CHMeO)_{y}-* où y représente un nombre entier de 5 à 35 ou un groupe
*-(CH₂CH₂O)ₓ-(CH₂CHMeO)_{y}-* où la somme x+y représente un nombre entier de 5 à 35 et
x et y sont supérieurs à zéro
et
(b) 1 à 20 % en poids d'au moins un polymère anionique, non réticulé, comprenant au moins un motif structural de formule (III) et au moins un motif structural de formule (IV), 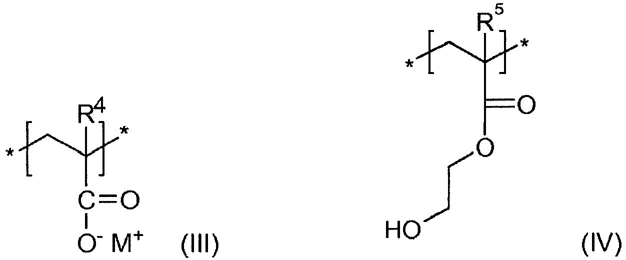 où
R⁴ et R⁵ représentent indépendamment l'un de l'autre un atome d'hydrogène ou un groupe méthyle,
M⁺ représente un cation physiologiquement acceptable
(c) 1 à 40 % en poids d'au moins un polymère fixant non ionique issu du groupe
(c1) des polymères non ioniques comportant au moins un élément structurel de formule (N5) 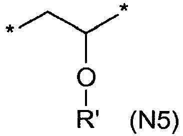 où R' représente un atome d'hydrogène, un groupe alkyle ou un groupe alcanoyl, de préférence un groupe acétyle,
(c2) des homopolymères et copolymères non ioniques de la N-vinylpyrrolidone,
(c3) des copolymères non ioniques de l'isobutène.

2. Agent selon la revendication 1 **caractérisé en ce que** A¹ représente un groupe *-(CH₂CH₂O)ₓ-* selon la formule (II), où x représente un nombre entier de 5 à 35, en particulier un nombre entier de 10 à 24.

3. Agent selon une des revendications 1 ou 2 **caractérisé en ce que** R² représente de préférence un groupe méthyle selon la formule (II).

4. Agent selon l'une quelconque des revendications 1 à 3 **caractérisé en ce que** le polymère anionique, non réticulé (b) contient en plus au moins un motif structural de formule (V), 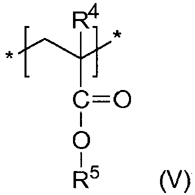 où
R⁴ représente un atome d'hydrogène ou un groupe méthyle,
R⁵ représente un groupe alkyle (en C₂ à C₄), en particulier le méthyle ou le n-butyle.

5. Agent selon l'une quelconque des revendications 1 à 4 **caractérisé en ce que** le polymère anionique, non réticulé (b) est un tétrapolymère linéaire d'acide méthacrylique, méthacrylate d'hydroxyéthyle, de méthacrylate de méthyle et d'acrylate de butyle.

6. Agent selon l'une quelconque des revendications 1 à 5 **caractérisé en ce qu'**il contient, comme polymère fixant non ionique (c) au moins un polymère issu du groupe
- polyvinylpyrrolidone,
- copolymères de N-vinylpyrrolidone et de vinylesters d'acides carboxyliques comportant 2 à 18 atomes de carbone, en particulier de N-vinylpyrrolidone et d'acétate de vinyle,
- copolymères de N-vinylpyrrolidone et de N-vinylimidazol et de méthacrylamide,
- copolymères de N-vinylpyrrolidone et de N-vinylimidazol et d'acrylamide,
- copolymères de N-vinylpyrrolidone avec N,N-dialkylamino(en C₁ à C₄)-alkylacrylamide(en C₂ à C₄),
- copolymères de N-vinylpyrrolidone avec N,N-dialkylamino(en C₁ à C₄)-alkylacrylamide(en C₂ à C₄).

7. Agent selon l'une quelconque des revendications 1 à 6 **caractérisé en ce qu'**il contient, par rapport à son poids -
- les polymères anioniques, amphipathiques, réticulés (a) dans une quantité de 1,5 à 17,5 % en poids, de préférence de 2 à 15 % en poids, de manière davantage préférée de 3 à 12,5 % en poids et en particulier de 5 à 10 % en poids,
- les polymères anioniques non réticulés (b) dans une quantité de 1,5 à 17,5 % en poids, de préférence de 2 à 15 % en poids, de manière davantage préférée de 2,5 à 12,5 % en poids et en particulier de 3 à 10 % en poids,
- les polymères fixants non ioniques (c) dans une quantité de 1,5 à 35 % en poids, de préférence de 2 à 30 % en poids, de manière davantage préférée de 3 à 25 % en poids et en particulier de 5 à 20 % en poids.

8. Agent selon l'une quelconque des revendications 1 à 7 **caractérisé en ce qu'**il contient les polymères anioniques, amphipathiques non réticulés (b) dans une quantité de 0,1 % en poids à 5,0 % en poids, de préférence de 0,2 % en poids à 5,0 % en poids, de manière particulièrement préférée de 0,1 à 2,0 % en poids, par rapport au poids de l'agent respectivement.

9. Agent selon l'une quelconque des revendications 1 à 8 **caractérisé en ce que** les polymères anioniques, amphipathiques, réticulés (a) et les polymères anioniques non réticulés (b) sont utilisés dans un rapport pondéral de 1 sur 5 à 5 sur 1, en particulier de 1 sur 2 à 5 sur 1, de manière particulièrement préférée de 1 sur 1,5 à 2 sur 1.

10. Utilisation d'un agent selon au moins une des revendications 1 à 9 pour la mise en forme temporaire des cheveux et/ou pour l'entretien des cheveux.

11. Procédé de traitement de fibres kératiniques, en particulier des cheveux humains, dans lequel un agent selon au moins une des revendications 1 à 9 est appliqué sur les fibres kératiniques.
